# EUROPEAN PATENT APPLICATION

(11) **EP 4 461 292 A1**
(43) Date of publication of application: **13.11.2024**
(21) Application number: 23172599.5
(22) Date of filing: 10.05.2023
(51) Int. Cl.: A61K 8/73, A61K 8/98, A61Q 19/08

(54) **TOPICAL PRODUCTS FOR USE ON INTACT SKIN OR DAMAGED SKIN FOR COSMETIC USE AND/OR AS A MEDICAL DEVICE**

(71) Applicant: Blast Research srl, 20121 Milan (IT)
(72) Inventor: CARAVA', Elena, 21044 Cavaria con Premezzo (IT); COSTA, Massimo, 20159 Milan (IT); GALGANO, Marta, 20157 Milan (IT); GIUNTINI, Ilaria, 22100 Como (IT); PASSI, Alberto, 26100 Cremona (IT)
(74) Representative: Zaccaro, Elisabetta

(57) **Abstract**

The present invention concerns the cosmetic field, and in particular relates to a cosmetic product and to its uses.

Specifically, the invention relates to a cosmetic product comprising a concentrate and sonicated hyaluronic acid, having a mean molecular weight in the range from 100kDa to 500kDa; wherein said concentrate comprises at least one growth factor and at least one cytokine.

The invention further relates to the use of the cosmetic product for promoting tissue regeneration, for improving skin texture, for reducing skin wrinkles and scars and for reducing skin irritation and blemishing.

## Description

### FIELD OF THE INVENTION

The present invention concerns the cosmetic field, and in particular relates to a cosmetic product and to its uses.

Specifically, the invention relates to a cosmetic product comprising a concentrate and sonicated hyaluronic acid, having a mean molecular weight in the range from 80kDa to 1200kDa; wherein said concentrate comprises at least one growth factor and at least one cytokine.

The invention further relates to the use of the cosmetic product for promoting tissue regeneration, for improving skin texture, for reducing skin wrinkles and scars and for reducing skin irritation and blemishing.

### STATE OF THE ART

Currently there is no remedy for maturing skin and treatments for skin that shows the signs of aging and/or wrinkles are temporary and suffer from drawbacks and side effects. Breakdown of skin strength and thickness is due to loss of collagen and elastic proteins present in the dermal layers, which may result in fine lines and wrinkles.

There are many surgical means available for treatment of facial wrinkles such as face-lifts, laser surgery, skin peels, and injection therapies. However, surgical methods must be repeated with time and may result in complications. Non-invasive remedies include topical formulations, however, none of these methods completely eliminate wrinkles, and require multiple, and often expensive treatments. Some topical formulations may act as irritants to the skin, to elicit wound healing responses, but do not successfully replenish the thinning skin with adequate proteins for treatment and/or prevention of age-related defects.

Cell culture medium is vital for *in vitro* cell growth and function and provides nutrients and growth factors such as essential amino acids, salts, vitamins, minerals, trace metals, sugars, lipids and nucleosides.

Many cell culture media have been formulated to allow the growth of a wide range of cell types and experimental applications. Cells are said to be "incubated" with a cell culture medium once they are put in contact with it.

A cell culture medium that is incubated with cells, is known as a "conditioned medium".

Conditioned medium differs from the original medium due to the fact that it contains many cellular metabolites and secreted proteins as well as a variety of the original components of the medium. Examples of the metabolites and proteins that are secreted into the medium by the cells during growth are growth factors, inflammatory mediators and other extracellular proteins, which may have beneficial biological effects on epidermal remodeling and have been of increased interest in the field of cosmetics.

The need and importance are however increasingly felt for more effective cosmetic products for the treatment and/or prevention of skin defects due to aging and environmental factors.

It is therefore object of the present invention the development of advantageous products for skin care that have an effect in the dermal layers and not only on the skin surface.

### SUMMARY OF THE INVENTION

In fact, without being bound to any theory, the inventors have surprisingly found that the problem can be solved with a cosmetic product comprising:
- a concentrate comprising at least one growth factor and at least one cytokine, secreted from a fibroblast cell culture, and
- sonicated hyaluronic acid, having a mean molecular weight in the range from 80kDa to 1200kDa;
wherein said concentrate is obtainable by a process comprising the steps of:
a. Providing a fibroblast cell culture in a cell culture medium;
b. Stimulating the release of at least one growth factor and at least one cytokine into the cell culture medium of step a. to obtain a conditioned cell culture medium;
c. Isolating the conditioned cell culture medium of step b. from the cell culture, to obtain an isolated conditioned cell culture medium;
d. Concentrating the isolated conditioned cell culture medium of step c. to obtain a concentrate.

The cosmetic product can be, for example, a topical formulation, active in the treatment and/or prevention of skin damage, wrinkles and/or other defects due to aging and environmental factors.

The cosmetic product may be formulated for preventing, reducing and/or eliminating wrinkles, frown lines, scarring and other skin conditions associated with aging, in addition to or in the alternative to using surgery, injectables, silicone or other products.

In a second aspect thereof, this invention provides the use of the cosmetic product for promoting tissue regeneration.

In a third aspect, the cosmetic product according to the invention can be used for improving skin texture.

In a fourth aspect the invention relates to the use of the cosmetic product for reducing skin wrinkles.

In a fifth aspect, the invention relates to the use of the cosmetic product for reducing scars.

In a sixth aspect, the herein described is the use of the cosmetic product for reducing skin irritation and blemishing.

### BRIEF DESCRIPTION OF THE DRAWINGS

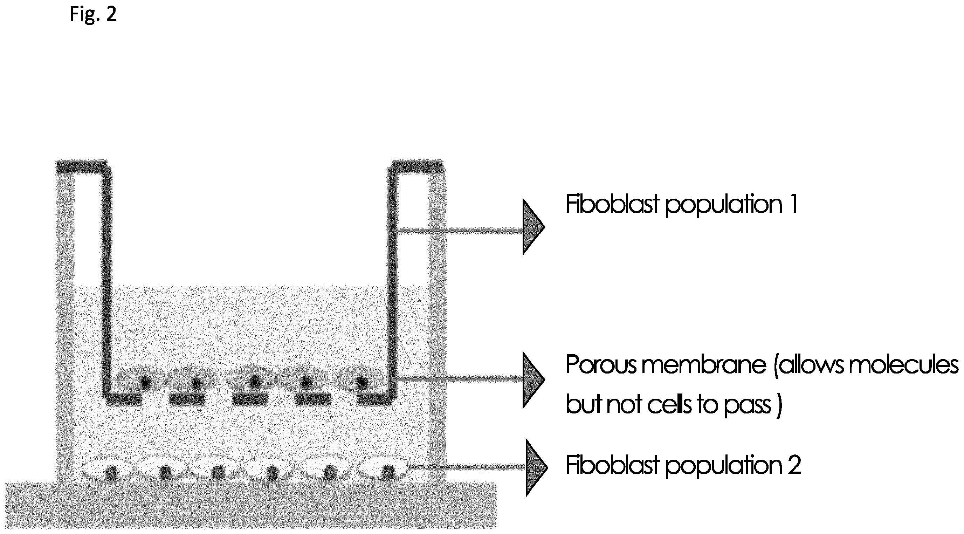

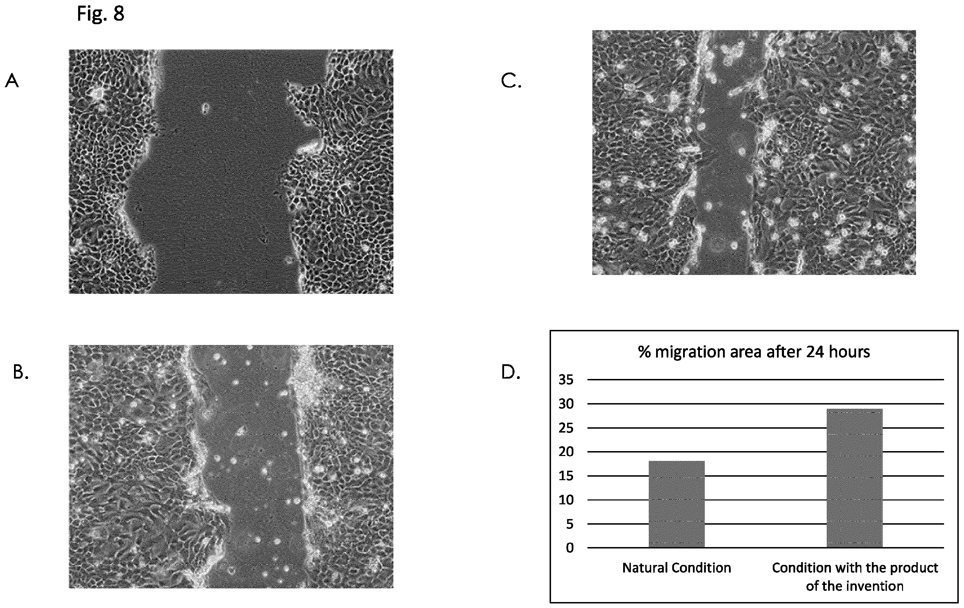

The characteristics and advantages of the present invention will be apparent from the detailed description reported below, from the Examples given for illustrative and nonlimiting purposes, and from the annexed Figures 1-8.
Figure 1: Electrophoresis gel to examine the protein content. CCM samples were loaded onto acrylamide gel (in 4-12% gradient): in position 4 we find lot 1 and in position 7 we find lot 2. Subsequently the gel was colored with silver color which allowed to highlight the proteins in brown.
Figure 2: shows an illustration of the co-culture assay in the traswell system as described in Example 6.
Figure 3: RT-PCR results. Bars correspond to gene expression level of HMGB1 and Collagen type 1 in fibroblasts: fibroblast population 1 (left), fibroblast population 2 (right) and the bar in the center corresponds to the fibroblast population 1 cultured in the transwell system that are in indirect contact with fibroblast population 2. Left and right bars are the control cells and central bars are treated cells. A. HMGB1 48h and B. Collagen type 1, Day 8.
Figure 4: Gel electrophoresis of HA samples after Stain-All staining. This dye allows hyaluronic acid to be identified in blue.
Figure 5: Gaussian distribution of HA sonicated fragments as described in Example 7. A. Cream and B.Serum
Figure 6: Stability test of HA. Electrophoresis on agarose gel and staining with Stain All. This dye allows hyaluronic acid to be identified in blue.
Figure 7: Fibroblast Scratch Test. Images acquired at 100x and graph of the results obtained at: A. T0: fibroblasts immediately after an injury, B. T24: natural cell migration after 24 hours if the skin is not treated and C. T24: cell migration after 24 hours if the skin is treated with cosmetic product of the invention in the 24 hours of incubation. D: graph of % migration area after 24h.
Figure 8: Scratch test on keratinocytes. Images acquired at 100x and graph of the results obtained over the 24 hours of incubation. Results obtained at: A. T0: keratinocytes immediately after an injury, B. T24: natural cell migration after 24 hours if the skin is not treated, C. T24: cell migration after 24 hours if the skin is treated with cosmetic product of the invention and D. graph of % migration area after 24h.

### DETAILED DESCRIPTION OF THE INVENTION

The present invention concerns a cosmetic product comprising:
- a concentrate comprising at least one growth factor and at least one cytokine, secreted from a fibroblast cell culture, and
- sonicated hyaluronic acid, having a mean molecular weight in the range from 80kDa to 1200kDa;
wherein said concentrate is obtainable by a process comprising the steps of:
a. Providing a fibroblast cell culture in a cell culture medium;
b. Stimulating the release of at least one growth factor and at least one cytokine into the cell culture medium of step a. to obtain a conditioned cell culture medium;
c. Isolating the conditioned cell culture medium of step b. from the cell culture, to obtain an isolated conditioned cell culture medium;
d. Concentrating the isolated conditioned cell culture medium of step c. to obtain a concentrate.

The resulting cosmetic product contains a mixture of growth factors and cytokines secreted by fibroblasts together with matrix proteins.

As used herein, the term "concentrate" or "Concentrated Conditioned Medium" or "CCM" refers to the product obtained after step d. of the process of the present invention. Example 4 describes a possible embodiment for obtaining the CCM by Tangential Flow Filtration.

As used herein, the term "sonicated Hyaluronic acid" or "HA" or "sonicated sodium Hyaluronate" refers to hyaluronic acid that has undergone the process of sonication, as exemplified in Example 7 and which comprises hyaluronic acid fragments having different sizes. Based on the product formulation, HA can be present as only sonicated HA or as a mixture of sonicated HA and non-sonicated low molecular weight HA. For example, a product can contain only sonicated HA having a molecular weight in the range of 1200 kDa-80 kDa, or a mixture of sonicated hyaluronic acid (in the range of 1200 kDa-80 kDa) with the addition of low molecular weight hyaluronic acid (HA-LMW), having a molecular weight in the range of 1000-400 kDa which has not been sonicated. Typically HA in the products of the invention has a size range from 1200 kDa to 80 kDa in varying amounts, for example in the amounts:
- 5 to 20%, preferably of about 9% fragments from 1200 to 800 kDa,
- 30 to 40%, preferably of about 32-33 % fragments from 800 to 400 kDa,
- 30 to 40%, preferably of about 34-35 % fragments from 400 to 200 kDa ; and
- 5 to 20%, preferably of about 15% of fragments from 200 to 80 kDa.

One of the most important characteristics of HA is represented by its molecular weight. Molecular weight determines HA's properties and is essential in determining the skin permeability of the molecule.

Morphologically, if the polymer is short, it unravels more easily and has an advantage in movement (medium/low molecular weight HA). The longer the polymer, the bulkier and slower it is (high molecular weight HA).

High molecular weight HA (over 500 kDa) can't be skin absorbed, and carries out its action at the epidermis level. In fact, when applied to the surface of the skin, high molecular weight HA, acts by forming a hydrated viscoelastic film. This film is permeable to air, so cutaneous respiration is not obstructed while the film "fixes" moisture to the skin surface. Indeed, the most important advantage of high molecular weight hyaluronic acid is its good water retention capacity, so once applied on the skin, HA mainly works as a film-forming polymer: it reduces water evaporation, with an occlusive-like action. Moreover, the HA film-forming action allows, by mechanical means, the diminution of skin evaporation and limits interaction with the environmental factors such as temperature, humidity, and UV radiation. In this way HA's protective barrier improves skin hydration and promotes local healing of superficial wounds.

Medium/low molecular weight HA are able to exert their function in the most deep layers of the skin, reaching the dermis. Being smaller they encounter fewer obstacles in crossing the epidermis and perform their functions in the dermis.

HA is a fundamental component of the dermis extracellular matrix because it stimulates the proliferation and migration of fibroblasts, endothelial cells, keratinocytes and promotes neoangiogenesis. Thanks to its action in the deeper layers of the skin, HA is able to keep the rate of deep epidermal hydration high, improving skin tone and elasticity. A contribution of medium/low molecular weight HA is essential to helping the physiological maintenance of the extracellular matrix in dermis.

In order for HA to have an action on both skin levels, a part of HA in the product of the invention is fragmented through sonication.

The "cosmetic product" as used herein, refers to a product that comprises two ingredients: hyaluronic acid (HA in the forms of both HA-LMW and the mixture of HA-LMW and sonicated HA, depending of the product formulation) and the CCM. Preferably the cosmetic product of the present invention can further comprise excipients suitable for the final formulation.

The two principal components: CCM and HA work in synergy to improve their individual potential.

Without being bound to any theory:
- the CCM acts as a stabilizing agent for HA
- HA interacting with CCM proteins allows them to pass through the epidermal barrier, favouring their action also in the dermis.

The combined action of CCM and HA in the cosmetic product, promotes the natural regeneration of the epidermis and dermis.

The concentrate is free from animal proteins and is obtained from the cultures of expanded fibroblasts in the process of the invention.

In an embodiment, the cosmetic product of the present invention is obtainable by a process, wherein said step b. of stimulating the release of at least one growth factor and at least one cytokine into the cell culture medium of said process is carried out by subjecting said fibroblast cell culture to a step of nutrient deprivation.

In a preferred aspect, at the end of the process, in step d., the concentrated conditioned can be sterile filtered.

In a further embodiment, the cosmetic product of the present invention is obtainable by a process, wherein said fibroblasts are isolated from a tissue sample, said tissue is preferably chosen from the group consisting of skin and umbilical cord.

For the isolation of cells from the chosen tissue, the skin biopsy undergoes digestion. As described in Example 1, tissues can be treated with a neutral protease, washed and subsequently digested with collagenase. The obtained cell suspension is then seeded and incubated in complete medium.

In a further embodiment therefore, the cosmetic product of the present invention is obtainable by a process, wherein said fibroblasts are isolated from said tissue sample by digestion with a protease, and subsequently with a collagenase.

In a further embodiment, the cosmetic product of the present invention is obtainable by a process, wherein said concentrate comprises a mixture of growth factors, cytokines and matrix proteins.

The quantification of the protein content of CCM can be performed by using the Bicinchoninic Acid (BCA) protein assay, also known as the Smith Assay, as shown in Example 5.

In a further embodiment, the cosmetic product of the present invention is obtainable by a process, wherein said concentrate comprises one or more of the growth factors, for example those listed in Table 1 which lists the main components of the fibroblast secretome. The cosmetic product of the present invention is obtainable by a process, wherein said concentrate comprises one or more of growth factor chosen from the group comprising: Vascular Endothelial Growth Factor (VEGF), Transforming Growth Factor β (TGF-β) and Fibroblast Growth Factors (FGFs), Platelet-Derived Growth Factor (PDGF), Granulocyte-Colony Stimulating Factor (GCSF), Keratinocyte Growth Factor (KGF), Transforming Growth Factor Beta-3 (TGF beta 3), Nerve Growth Factor Beta (NGF beta), Sirtuin 1 (SIRT 1), Sirtuin 2 (SIRT2), Tissue Inhibitor for Metalloproteinase 1 (TIMP 1), Macrophage Colony Stimulating Factor 1 (M-CSF or CSF 1), Stem Cell Factor (SCF), Insulin-Like Growth Factor (IGF-1), Insulin-Like Growth Factor 2 (IGF 2), Interleukin 10 (IL-10) and Hepatocyte Growth Factor (HGF),.

**Table 1**

| **Native protein** | **Category** | **Functions** | Reference |
|---|---|---|---|
| Transforming Growth Factor Beta-3 (TGF beta 3) | SKIN CONDITIONIN G | Cytokine involved in cell differentiation, cell adhesion and the formation of the extracellular matrix. Controls wound healing by regulating skin and dermis cells.TGF-β 3 has a role in orofacial development and has a potent anti-scarring effect | Le M, Naridze R, Morrison J, Biggs LC, Rhea L, Schutte BC, Kaartinen V, Dunnwald M. Transforming growth factor Beta 3 is required for excisional wound repair in vivo. PLoS One. 2012;7(10):e48040. doi: 10.1371/journal.pone.0048040. Epub 2012 Oct 26. PMID: 23110169; PMCID: PMC3482237. |
| Hepatocyte Growth Factor (HGF) | ANTIOXIDANT , SKIN CONDITION IN G, HAIR CONDITIONIN G | Hepatocyte growth factor (HGF) is produced by stromal and mesenchymal cells, and it stimulates epithelial cell proliferation, motility, morphogenesis and angiogenesis in various organs | Kato, T. (2017). Biological roles of hepatocyte growth factor-Met signaling from genetically modified animals (Review). Biomedical Reports, 7, 495-503. https://doi.org/10.3892/br.2017.1001 |
| Keratinocyte Growth Factor (KGF or FGF-7) | SKIN CONDITIONIN G | KGF enhances keratinocyte proliferation and migration, but delays differentiation. The expression of KGF is strongly up-regulated in a wounded dermis, and KGF enhances the wound closure suggesting its importance in the wound healing process. KGF has been shown to protect epithelial cells from the toxic effects of reactive oxygen species | Susanna Karvinen, et al. Keratinocyte Growth Factor Stimulates Migration and Hyaluronan Synthesis in the Epidermis by Activation of Keratinocyte Hyaluronan Synthases 2 and 3. Journal of Biological Chemistry. Volume 278, Issue 49, 2003. Pages 49495-49504. ISSN 0021-9258. https://doi.org/10.1074/jbc.M310445 200. Hans-Dietmar Beer, et al. Expression and Function of Keratinocyte Growth Factor and Activin in Skin Morphogenesis and Cutaneous Wound Repair, Journal of Investigative Dermatology Symposium Proceedings. Volume 5, Issue 1, 2000. Pages 34-39. https://doi.org/10.1046/j.1087-0024.2000.00009.x. |
| Fibroblast Growth factor 10 (FGF10) | SKIN CONDITIONIN G | FGF10 promotes fibroblast migration which is important in the promotion of wound repair | Song YH, Zhu YT, Ding J, Zhou FY, Xue JX, Jung JH, Li ZJ and Gao WY: Distribution of fibroblast growth factors and their roles in skin fibroblast cell migration. Mol Med Rep 14: 3336-3342, 2016 |
| Nerve Growth Factor Beta (NGF beta) | HAIR CONDITIONIN G, SKIN PROTECTING | Nerve growth factor (NGF) is a polypeptide which, in addition to its effect on nerve cells, is believed to play a role in inflammatory responses and in tissue repair. It plays important roles in tissue remodeling during the would healing process of the skin | Micera A, et al. Nerve growth factor displays stimulatory effects on human skin and lung fibroblasts, demonstrating a direct role for this factor in tissue repair. Proc Natl Acad Sci U S A. 2001 May 22;98(11):6162-7. doi: 10.1073/pnas.101130898. Masahiko Mori,et al.The functions of nerve growth factor and nerve growth factor receptor in wound healing. 2012 Volume 16 Issue 3+4 Pages 51-65. https://doi.org/10.3353/omp.16.51 |
| Sirtuin 1 (SIRT 1) | ANTIOXIDANT , HAIR CONDITION IN G, SKIN CONDITIONIN G | Sirtuins are implicated in the process of aging and photoaging. The level of SIRT1 changes expression profile depending on the age of the person (the level decreased with age). Sirtuin 1 plays a key role in epigenetic modification of proteins, histones, and chromatin by which regulates the expression of genes implicated in the oxidative stress response, inflammation, and apoptosis. It is use as an active ingredient of antiaging cosmetic products. | Bielach-Bazyluk A, Zbroch E, Mysliwiec H, Rydzewska-Rosolowska A, Kakareko K, Flisiak I, Hryszko T. Sirtuin 1 and Skin: Implications in Intrinsic and Extrinsic Aging-A Systematic Review. Cells. 2021 Apr 6;10(4):813. doi: 10.3390/cells10040813. PMID: 33917352; PMCID: PMC8067363. Garcia-Peterson L, M, Wilking-Busch M, J, Ndiaye M, A, Philippe C, G, A, Setaluri V, Ahmad N: Sirtuins in Skin and Skin Cancers. Skin Pharmacol Physiol 2017;30:216-224. doi: 10.1159/000477417 |
| Sirtuin 2 (SIRT2) | ANTIOXIDANT , HAIR CONDITION IN G, SKIN CONDITIONIN G | As SIRT 1, SIRT 2 is implicated in aging as well as in UV damage and oxidative stress responses | (the same of Sirtuin 1) |
| Tissue Inhibitor for Metalloproteina se 1 (TIMP 1) | SKIN CONDITIONIN G, SKIN CONDITIONIN G-EMOLLIENT, SKIN PROTECTING | The tissue inhibitors of metalloproteinases (TIMPs) are the primary endogenous inhibitors of matrix metalloproteinases (MMPs). TIMP-1 plays a key role in maintaining the balance between extracellular matrix breakdown and remodeling. | Elianne A. Koop, et al. CHAPTER 7 - TUMOR VASCULATURE AS A TARGET, Anticancer Drug Development, Academic Press, 2002, Pages 123-cp2. K.J. Leco, et al. MATRIX METALLOPROTEINASE INHIBITORS, Encyclopedia of Respiratory Medicine, Academic Press, 2006, Pages 9-18. https://doi.org/10.1016/B0-12-370879-6/00243-X. https://doi.org/10.1016/B978-012072651-6/50008-5. |
| Macrophage Colony Stimulating Factor 1 (M-CSF or CSF 1) | SKIN PROTECTING | Macrophage colony-stimulating factor (M-CSF) is a hematopoietic growth factor that regulates the proliferation, differentiation, and functional activation of monocytes. It promotes the differentiation of myeloid progenitors into heterogeneous populations of monocytes, macrophages, dendritic cells, and bone-resorbing osteoclasts. As a treatment, CSF-1 has therapeutic potential in tissue repair. | David A. Hume, Therapeutic applications of macrophage colony-stimulating factor-1 (CSF-1) and antagonists of CSF-1 receptor (CSF-1R) signaling. Blood (2012) 119 (8): 1810-1820 https://doi.org/10.1182/blood-2011-09-379214 |
| Platelet Derived Growth Factor (PDGF) | HUMECTANT, SKIN CONDITIONIN G | Platelet-derived growth factor (PDGF) is a potent activator for cells of mesenchymal origin. PDGF stimulates chemotaxis, proliferation, and new gene expression in monocytes-macrophages and fibroblasts in vitro, cell types considered essential for tissue repair | Pierce GF, Mustoe TA, Altrock BW, Deuel TF, Thomason A. Role of platelet-derived growth factor in wound healing. J Cell Biochem. 1991 Apr;45(4):319-26. doi: 10.1002/jcb.240450403. PMID: 2045423. |
| Stem Cell Factor (SCF) | ANTIOXIDANT , HAIR CONDITION IN G, SKIN CONDITIONIN G | Stem cell factor (SCF) is a growth factor that binds to the receptor tyrosine kinase and leads to activation of multiple signal transduction factors. SCF has a role in the differentiation, survival and self-renewal of hematopoietic stem cells | Atef A, El-Rashidy MA, Abdel Azeem A, Kabel AM. The Role of Stem Cell Factor in Hyperpigmented Skin Lesions. Asian Pac J Cancer Prev. 2019 Dec 1;20(12):3723-3728. doi: 10.31557/APJCP.2019.20.12.3723 |
| Vascular Endothelial Growth Factor A (VEGF-A) | SKIN CONDITIONIN G | Vascular Endothelial Growth Factor A regulate angiogenesis and vasculogenesis. | Holmes, D.I., Zachary, I. The vascular endothelial growth factor (VEGF) family: angiogenic factors in health and disease. Genome Biol 6, 209 (2005). https://doi.org/10.1186/gb-2005-6-2-209 |
| Insulin-Like Growth Factor (IGF-1) | SKIN CONDITIONIN G, SKIN PROTECTING | Insulin Growth Factor 1 stimulates these fibroblasts to proliferate and provide loose connective tissue. It acts on epidermal keratinocytes stimulating their proliferation. | Farag AGA, Abdu Allah AMK, El-Rebey HS, Mohamed Ibraheem KI, Mohamed ASED, Labeeb AZ, Elgazzar AE, Haggag MM. Role of insulin-like growth factor-1 in skin tags: a clinical, genetic and immunohistochemical study in a sample of Egyptian patients. Clin Cosmet Investig Dermatol. 2019 Apr 26;12:255-266. doi: 10.2147/CCID.S192964. PMID: 31118729; PMCID: PMC6503204. |
| Acidic Fibroblast Growth Factor 1 (FGF 1 or aFGF) | - | FGFs have the biological activity of stimulating the proliferation of fibroblasts and angiogenesis. They have a important role in repair and remodeling of the dermis during the skin anti-aging process and can act on cell regeneration and repair processes | de Araújo R, Lôbo M, Trindade K, Silva D, F, Pereira N: Fibroblast Growth Factors: A Controlling Mechanism of Skin Aging. Skin Pharmacol Physiol 2019;4:275-282. doi: 10.1159/000501145 |
| Interleukin 10 (IL-10) | HUMECTANT, SKIN CONDITIONIN G | Interleukin 10 (IL-10) is a cytokine with potent anti-inflammatory properties that plays a central role in limiting host immune response to pathogens, thereby preventing damage to the host and maintaining normal tissue homeostasis. In skin, it facilitates the regenerative healing regulating the extracellular matrix and the cellular function of fibroblasts and endothelial progenitor cells. | King A, Balaji 5, Le LD, Crombleholme TM, Keswani SG. Regenerative Wound Healing: The Role of Interleukin-10. Adv Wound Care (New Rochelle). 2014 Apr 1;3(4):315-323. doi: 10.1089/wound.2013.0461. PMID: 24757588; PMCID: PMC3985521. |
| Transforming Growth Factor Beta 1 (TGF b1) | SKIN CONDITIONIN G | Transforming Growth Factor-β (TGF-β) is a key player in cell proliferation, differentiation and apoptosis. It's impicated in wound healing, angiogenesis and immunoregulation. | Prud'homme, G. Pathobiology of transforming growth factor β in cancer, fibrosis and immunologic disease, and therapeutic considerations. Lab Invest 87, 1077-1091 (2007). https://doi.org/10.1038/labinvest.370 0669 |
| Basic Fibroblast Growth Factor (FGF 2 or bFGF) | SKIN CONDITIONIN G | FGFs have the biological activity of stimulating the proliferation of fibroblasts and angiogenesis. They have a important role in repair and remodeling of the dermis during the skin anti-aging process and can act on cell regeneration and repair processes. bFGF is clinically widely accepted and used in accelerating wound healing. | de Araújo R, Lôbo M, Trindade K, Silva D, F, Pereira N: Fibroblast Growth Factors: A Controlling Mechanism of Skin Aging. Skin Pharmacol Physiol 2019;4:275-282. doi: 10.1159/000501145. Akita 5, Akino K, Hirano A. Basic Fibroblast Growth Factor in Scarless Wound Healing. Adv Wound Care (New Rochelle). 2013 Mar;2(2):44-49. doi: 10.1089/wound.2011.0324. PMID: 24527324; PMCID: PMC3623580. |
| Insulin-Like Growth Factor 2 (IGF 2) | SKIN CONDITIONIN G | Insulin-Like Growth Factor 2 (IGF 2) regulate cell proliferation, growth, migration, differentiation and survival. | Bergman D, Halje M, Nordin M, Engström W: Insulin-Like Growth Factor 2 in Development and Disease: A Mini-Review. Gerontology 2013;59:240-249. doi: 10.1159/000343995 |

From the studies carried out so far we can say with certainty that FGF2 and type 1 collagen are present in the CCM, as can be seen from Table 2A and 2B:

**Table 2A:**

| **CCM batches** | **Collagen type 1 pg/ml** |
|---|---|
| Lot 1 | 357,6 |
| Lot 2 | 456,7 |
| Lot 3 | 404,3 |
| Lot 4 | 387,5 |

**Table 2B:**

| **CCM batches** | **FGF2 pg/ml** |
|---|---|
| Lot 1 | 64,7 |
| Lot 2 | 54,6 |
| Lot 3 | 36,4 |

The Concentrated Conditioned Medium (CCM) is a conditioned medium obtained from fibroblast culture without animal origin component. At the end of the process of the present invention, steps c. and d., the collected concentrated conditioned medium was optionally sterile filtered. The final product contained a mixture of growth factors and cytokines secreted by fibroblasts together with matrix proteins that are useful for tissue regeneration.

The CCM contains about 10-50 ug/ml of proteins with different molecular weights. Table 3 shows the quantification of the protein content of CCM with micro BCA.

**Table 3:**

| **CCM batches** | **OD1** | **OD2** | **Protein conc. (µg/ml)** | **Average-blank** |
|---|---|---|---|---|
| Lot 1 | 0.1806 | 0.1800 | 0.066400006 | 11 |
| Lot 2 | 0.2673 | 0.2772 | 0.1578500005 | 73 |
| Lot 3 | 0.1649 | 0.1671 | 0.052100003 | 15 |
| Lot 4 | 0.2670 | 0.2802 | 0.159699999 | 74 |

This heterogeneous group of proteins is referred to in literature as the fibroblast secretome. Inside there are innumerable proteins, some of which are still unidentified.

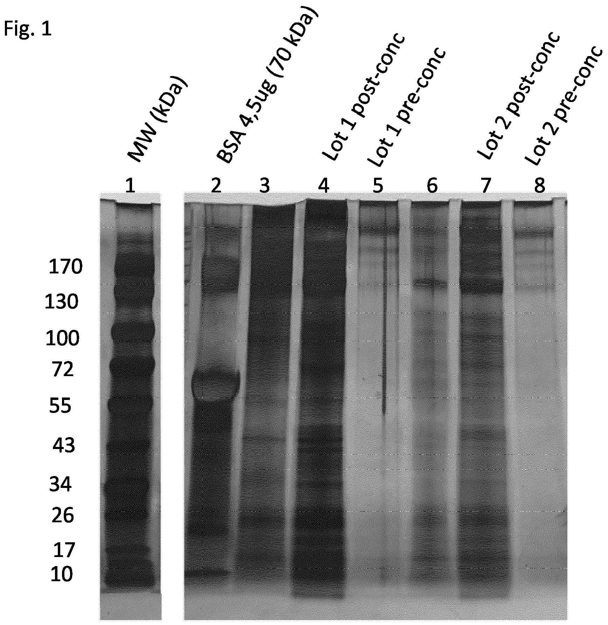

Figure 1 shows the result of an electrophoresis of the fibroblast protein content.

The main molecules present in the fibroblast secretome and consequentially in the CCM are: Vascular Endothelial Growth Factor (VEGF), Transforming Growth Factor β (TGF-β) and Fibroblast Growth Factors (FGFs). These last factors play a relevant role in the deposition of collagen in the extracellular matrix and in antiaging therapy because the collagen and elastin synthesis are responsible for skin elasticity and resistance, characteristics that are diminished with skin aging.

Other growth factors like Platelet-Derived Growth Factor (PDGF), Granulocyte-Colony Stimulating Factor (GCSF), Keratinocyte Growth Factor (KGF), and Hepatocyte Growth Factor (HGF) are implicated in dermal remodelling by stimulating synthesis of new collagen, elastin, glycosaminoglycans, and by mediating angiogenesis.

Without being bound to any theory, in order to clarify the mechanism of action of the CCM, a co-culture test was carried out and allowed to verify whether the components of the secretome were released from the fibroblasts into the culture medium and whether these influenced a second population of fibroblasts. The essay wanted to mimic a physiological condition where different populations of fibroblasts reside within the same tissue as the skin.

The behavior of fibroblasts was studied using a transwell system. It was possible to study the indirect interaction of two different cell populations incubated together that have only the culture medium in common (Example 6).

In a further embodiment, the cosmetic product of the present invention is obtainable by a process, wherein said sonicated hyaluronic acid comprises sonicated hyaluronic acid fragments having a size from 1200 kDa to 80 kDa in varying amounts, for example in the amounts of about 9% fragments from 1200 to 800 kDa, of about 32-33 % fragments from 800 to 400 kDa, of about 34-35 % fragments from 400 to 200 kDa and of about 15% of fragments from 200 to 80 kDa.

One of the most important characteristics of HA is represented by its molecular weight. Molecular weight determines the HA's properties and is essential in determining the skin permeability of the molecule.

Morphologically, if the polymer is short, it unravels more easily and has an advantage in movement (medium/low molecular weight HA). The longer the polymer, the bulkier and slower it is (high molecular weight HA).

High molecular weights HA (over 500 kDa) can't be skin absorbed and they carry out their action at the level of the epidermis. In fact, when applied to the surface of the skin, high molecular weight HA, acts forming a hydrated viscoelastic film. This film is permeable to air, so cutaneous respiration is not obstructed while the film "fixes" moisture to the skin surface. Indeed, the most important advantage of high molecular weight hyaluronic acid is its good water retention capacity, so once applied on the skin, HA mainly works as a film-forming polymer: it reduces water evaporation, with an occlusive-like action. Moreover, the HA film-forming action allows, by mechanical means, the diminution of skin evaporation and limits interaction with the environmental factors such as temperature, humidity, and UV radiation. In this way HA's protective barrier improves skin hydration and promotes local healing of superficial wounds. Medium/low molecular weight HA is able to exert its function in the most profound states of the skin, reaching the dermis. Being smaller they encounter fewer obstacles in crossing the epidermis and performs its functions in the dermis.

HA is a fundamental component of the dermis extracellular matrix because it stimulates the proliferation and migration of fibroblasts, endothelial cells, keratinocytes and promotes neoangiogenesis. Thanks to its action in the deeper layers of the skin, HA is able to keep the rate of deep epidermal hydration high, improving skin tone and elasticity. A contribution of medium/low molecular weight HA is essential to helping the physiological maintenance of the extracellular matrix in dermis.

In order for HA to have an action on both levels of the skin (outer layer and inner layer), a part of the HA in the cosmetic product of the present invention is fragmented through sonication. The instrument used for HA sonication was Sonicated Sonics Vibracell model VCX 130 S.N. 40225L at the 100% frequency for 10 minutes with intervals of 10 seconds ON and 10 seconds OFF. Other instruments may be used to obtain the resulting fragments in different size ranges, what needs to be taken into account is the instrument settings that need to be adapted to the scope.

The fragmentation process takes place through physical and non-chemical agents in order not to have reaction intermediates or processing residues that could be harmful (Example 7).

In a further embodiment, the cosmetic product of the present invention is obtainable by a process, wherein said cosmetic product is in the form of a cream or a serum and further comprises cosmetically acceptable excipients.

The cosmetic product can be formulated as a cosmeceutical facial cream, lotion, and/or serum for topical application, with or without additional growth factors, peptides, and/or other proteins and biologically active substances. In addition to the active ingredients, the product can comprise cosmetically acceptable excipients such as emollients, water-binding agents. Moisturizers, vitamins, anti-oxidants, anti-irritants and soothing agents. The cosmetic product of the present invention has surprisingly been shown positive effects in the promotion of the natural regeneration of the dermis in different cases such as: post-laser and post-radiotherapy treatments, scars, wrinkles, various types of irritation (redness and sun irritation).

In a second aspect the invention thus provides the use of the cosmetic product for promoting tissue regeneration.

In a third aspect, the cosmetic product according to the invention can be used for improving skin texture.

In a fourth aspect the invention relates to the use of the cosmetic product for reducing skin wrinkles.

In a fifth aspect, the invention relates to the use of the cosmetic product for reducing scars.

In a sixth aspect, the herein described is the use of the cosmetic product for reducing skin irritation and blemishing.

Various embodiments and aspects of the present invention as delineated hereinabove and as claimed in the claims section below find experimental support in the following examples.

### EXAMPLES

Reference is now made to the following examples, which together with the above descriptions illustrate some embodiments of the invention.

Tissue samples were obtained with prior informed consent sought from the donors.

Production of the Concentrated Conditioned Medium (CCM) involved the selection of a suitable skin or umbilical cord donor (tissue discarded) in accordance with the European Directive on the donation, procurement, testing, processing, preservation, storage and distribution of human tissues and cells (2004/23/EC, 2006/17/EC and subsequent updates and national transpositions).

All human tissue samples were preserved in a specific transport medium, and were sent by a qualified courier with controlled transport at 2-8°C to an authorized cell factory. Upon arrival, after verifying the integrity of the packaging, the serological report and the temperature trace, the tissue was subjected to digestion through specific GMP grade enzymes (neutral protease, collagenase and hyaluronidase)

The enzymes used for the digestion of the tissue were usually lyophilized and then reconstituted according to the manufacturer's instructions.

### Example 1.: CELL ISOLATION FROM SKIN

### Neutral protease digestion

For digesting tissue pieces of the size of 1 cm²:
7 mL of digestion solution were prepared by adding a volume of Neutral Protease equal to 6 DMCU (1.2 mL) and brought to volume with Hanks balanced salt solution (HBSS).14 µL of the 1M CaCl₂ solution were added to make a 2mM solution. 1DMC Unit catalyzes the cleavage of 1 µmol peptide bond from dimethylcasein per minute at 25 °C, pH 7.0, expressed in terms of newly formed terminal amino groups, determined with TNBS1. Before proceeding with Neutral protease digestion, 2 washes were carried out with a solution of DPBS with antibiotic (pen-strep) and 2 washes with only Dulbecco's phosphate buffered saline (DPBS)

After the washes the skin sample was inserted into a tube containing the digestion solution and the sample is incubated at 2-8°C overnight.

For tissue samples having a larger size, sections were cut with a scalpel into smaller pieces and proportional volume was used for the above digestion method.

### Dermis/epidermis separation, collagenase digestion and seeding in flask

After the removal of the neutral protease the sample was washed with DPBS, transferred into a petri dish to perform dermis / epidermis separation. After the removal of epidermis, the dermis was fragmented into small pieces. The fragments were inserted into a tube prepared with 10 mL of Collagenase solution (0.3 PZU/mL in HBSS) and 20 µL of a 1M solution of CaCl₂ (final 2mM solution). The sample was incubated for 2h at 37°C in an incubator. PZU: 1U according to Wünsch catalyzes the hydrolysis of 1 µmol 4-phenylazobenzyloxycarbonyl-L-prolyl-L-leucylglycyl-L-prolyl-D-arginine per minute at 25 °C, pH 7.1.

At the end of the 2h digestion, the digestion solution was filtered with a 100 µm filter. The filter was washed with 10 mL of HBSS, centrifuged at 1500 rpm for 10 min.

The supernatant was discarded, the pellet resuspended in 10 mL complete medium (CM) and the total cell suspension seeded in T25 flasks and incubated in the incubator at 37°C and 5% CO₂ for 2-4 days.

### Materials:

### a. Preparation of Neutral Protease:

The lyophilized enzyme was initially resuspended in order to obtain 100 DMCU/mL and then further diluted to 5 DMCU / mL. The solution was sterile filtered and divided into 1.5 ml aliquots. The vials were identified with the product name, lot, concentration, and expiration date (1 year after reconstitution).

### b. Preparation of Collagenase:

The lyophilized enzyme was initially resuspended 1 mL in order to obtain the stock solution (PZU/mL according to the concentration indicated in the certificate of analysis). To prepare a working solution, the stock solution was diluted and sterile filtered in order to achieve aliquots with 3PZU concentration. The solution must be constantly stored on ice until use. The vials were identified with the product name, lot, concentration, and expiration date (1 year after reconstitution).

### c. Preparation of the complete medium (CM):

The culture medium consisting of DMEM, Glutamine, Sodium Pyruvate, and Fetal Bovine Serum (irradiated and EDQM-certified) was identified as: the Complete Medium (CM) and its formula was as follows:
- 500 mL DMEM (high glucose)
- 50 mL FBS
- 10 mL Glutamine 200 mM
- 5 mL Sodium Pyruvate 1M.

All medium components were sterile, preparation was performed under a laminar flow hood (grade A) with a grade B surrounding, components are added sterilely into the bottle containing DMEM.

### Example 2.: CELL ISOLATION FROM UMBILICAL CORD

### Hyaluronidase and collagenase digestion and seeding in flask

The cord sample was washed with DPBS, transferred into a petri dish to perform separation in small pieces and then crushed into a fine mass. The mass of tissue was inserted into a 50 mL tube and an equal volume of DPBS was added, with a quantity of hyaluronidase, collagenase equal to 1% of the final volume and an amount of calcium gluconate equal to 0.04 % of a 10% solution of calcium gluconate. The sample was incubated for 2h at 37°C in an incubator.

At the end of digestion, the sample was diluted with DPBS (1:1), centrifuged at 300g for 10 seconds to sediment all the undigested debris and the digestion solution was passed through a 1 mm strainer and then with a 100 µm filter. The solution was centrifuged at 450g rpm 7 min.

The supernatant was discarded, the pellet resuspended in 25 mL complete medium (CM) and the total cell suspension seeded in a T 150 flask and incubated in an incubator at 37°C and 5% CO₂ for 2-4 days.

### Materials:

### a. Preparation of Hyaluronidase:

The lyophilized enzyme was resuspended in order to obtain a solution of 10 KU/mL. The solution was sterile filtered and divided into 1 ml aliquots. The vials were identified with the product name, lot, concentration, and expiration date (1 year after reconstitution)

### b. Preparation of Collagenase:

The lyophilized enzyme was resuspended in order to obtain a solution of 50mg/ml. The solution was sterile filtered and divided into 1 ml aliquots.

The vials were identified with the product name, lot, concentration, and expiration date (1 year after reconstitution).

### c. Preparation of the complete medium (CM):

The culture medium consisted of DMEM low glucose, Fetal Bovine Serum (irradiated and EDQM-certified), human platelet lysate (HPL), heparin. The Complete Medium (CM) formula was as follows:
- 500 mL DMEM (low glucose)
- 15 mL FBS
- 15 mL HPL
- 2.5 mL heparin (1000U/mL)

All medium components were sterile, preparation was performed under a laminar flow hood (grade A) with a grade B surrounding, components were added sterilely into the bottle containing DMEM.

### Example 3.: CELLULAR EXPANSION AND INOCULATION IN BIOFACTORY

### EXPANSION:

After the isolation of fibroblasts, the expansion scale up was identical for the two different tissues of Examples 1 and 2, only the medium for cell expansion changes. Medium for derma fibroblasts is reported in Example 1 while that for cord fibroblast is in Example 2.

The starvation medium is the same for the two tissue samples.

When the cells reach confluence of about 80 % observable under the microscope the fibroblasts are detached from the flask using trypsin, counted and seeded into new T150 flasks. A series of expansions, from 1 T150 to 3 T150 and from 3 to 12 T150 allow to obtain the quantity of cells necessary to inoculate from 2 -3 biofactories with 1L of complete medium. All the steps were carried out maintaining the same culture conditions (37°C and 5 % CO₂) by changing the medium every 2-4 days until the confluence is reached.

### STARVATION:

Upon reaching confluence in the biofactories, the complete medium was removed (DMEM, Glutamine, Sodium Pyruvate, FBS) and after 2 washing steps with DPBS, 1L of incomplete medium (DMEM without phenol red, Glutamine, Sodium Pyruvate) was added to each biofactory for 48h. The nutrient deprivation for 48h had the effect of stimulating the release of regenerative factors in the culture medium. After this incubation the medium was collected in sterile bottles and centrifuged to remove cellular debris. The supernatant was filtered with 0.22 filter systems (500 ml), the bottles identified with the code and the batch freezed at -20 ° C.

### Example 4.: CONCENTRATION OF THE CONDITIONED MEDIUM BY TANGENTIAL FLOW FILTRATION (TFF)

This type of concentration allows to speed up the process and to use greater quantities of supernatant in the order of liters.

The TFF concentration process was performed by setting the following operating parameters:
Phase I: 10X concentration
Phase II: diafiltration 10 volumes of buffer (NaCl 0.9%)
Phase III: 20X concentration (final)
Transmembrane pressure (TMP): 0.3 bar
Flow: 500ml / min
Cut-off of 10 kDa

The set parameters allowed to have an average permeate flow of about 54 ml / min or about 20 L / m² / h and a shear stress of about 2300/2400 s-1. Phase I of concentration, phase II of diafiltration and phase III of concentration last respectively 32 ', 30' and 2 '. The system was then emptied to recover all the product in the chosen bag / container and filtered through 0.22 mm syringe filters and divided into aliquots. All aliquots are frozen at -20 ° C.

### Example 5.: RELEASE TEST FOR THE CONCENTRATED CONDITIONED MEDIUM: PROTEIN DETERMINATION

The quantification of the protein content of CCM is performed using the Bicinchoninic Acid (BCA) protein assay. The Bicinchoninic Acid (BCA) protein assay, also known as the Smith Assay, is a highly sensitive colorimetric assay that is compatible with solubilized protein solutions.

The amount of protein present in a solution could be quantified by measuring the absorption spectra and comparing with protein solutions with known concentrations.

A standard albumin curve was used in the test. After 2 hours of incubation the samples were read at 562 nm. The average 562 absorbance measurement of the blank standards was subtracted from the average 562 absorbance of each standard and of each sample. A standard curve was plotted by placing the concentration of the standards on the X axis and on the Y axis the absorbance.

The obtained standard curve was used to determine the protein concentration of each unknown sample.

### Example 6.: CONDITIONED MEDIUM CHARACTERIZATION

The behavior of fibroblasts, to study the indirect interaction of two different cell populations was studied using a traswell system that is a co-cultured method. With this system, the cells were incubated together and only had the culture medium in common. The transwell system provided an external chamber where, on the bottom of which one population of fibroblasts were grown, and an internal chamber with a porous membrane on which a second population of fibroblasts were placed.

The two populations were not in direct contact and the transwell system allowed the passage of molecules released by the cells in the upper compartment towards the cells of the lower compartment.

Tests were performed both with two population of "normal" fibroblast (called population 1 and population 2) that have not been frozen and with one population "normal" and one population of "Frozen BLAST Fibroblast" (frozen in a Tyrode modified solution).

After 48 hours and 8 days, the expression level of two markers linked to tissue regeneration was analyzed: HMGB1 and Collagen type 1.

Only the results of normal fibroblast population 1 and 2 are listed in the document.

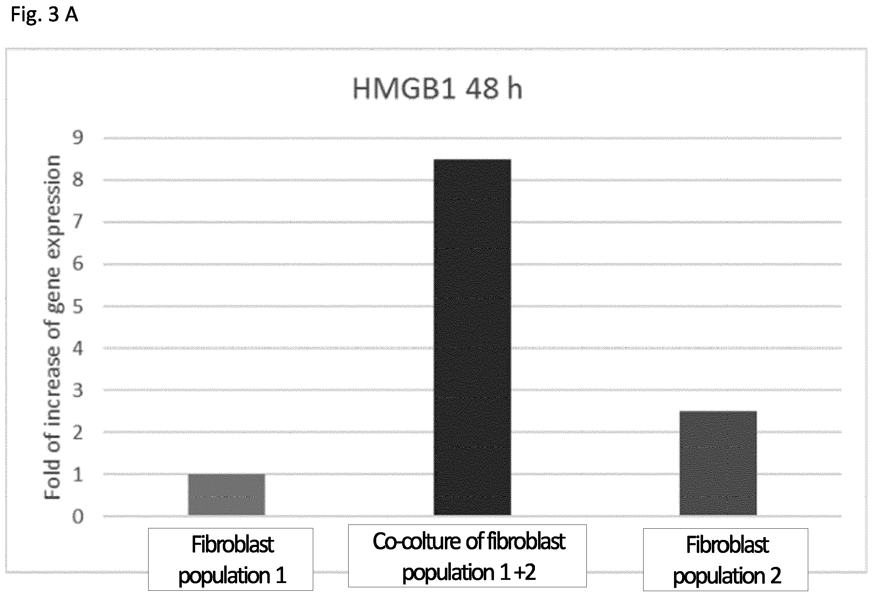

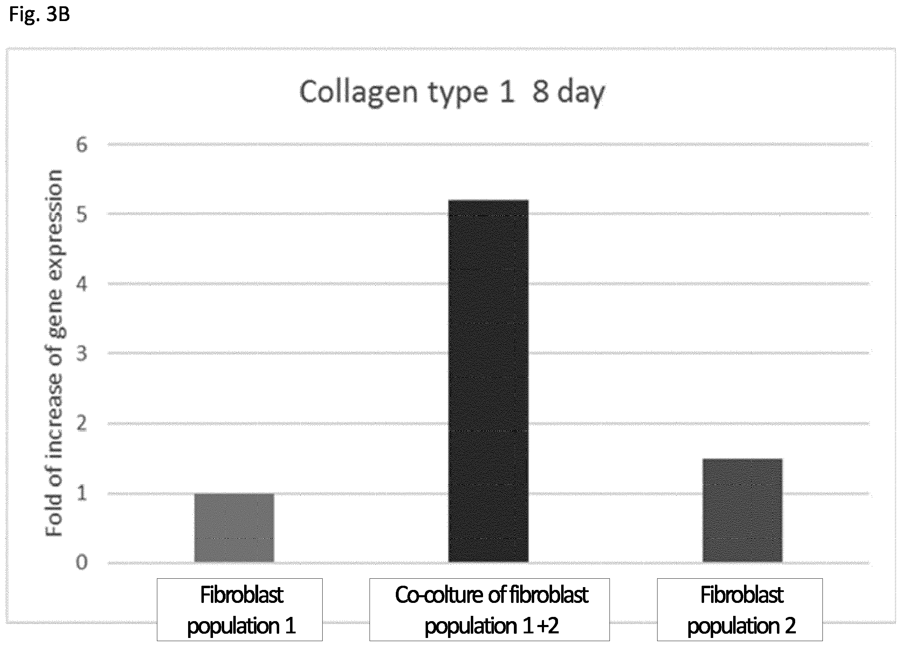

When the population 1 is co-cultured with the population 2 there is an increase production of the signal molecule HMGB1 after 48 hours and of Collagen type 1 after 8 days in fibroblasts population 1. This shows that the fibroblasts release factors into the culture medium that can stimulate the surrounding environment through a paracrine effect (Figure 3).

Similar results have been found in "normal" fibroblast and "frozen BLAST fibroblast" co-culture assay.

### Example 7.: HYALURONIC ACID SONICATION METHOD

The fragmentation process takes place through physical and non-chemical agents in order not to have reaction intermediates or processing residues that could be harmful. 0,25% of High Molecular Weight hyaluronic acid (HMW-HA of about 2.2000-1.800 kDa) in physiological solution was sonicated at the 100% frequency for 10 minutes with intervals of 10 seconds ON and 10 seconds OFF (Sonicated Sonics Vibracell model VCX 130 S.N. 40225L). The time, the setting and the frequency chosen for the sonication method of HA are also important to avoid the formation of small fragments with a pro-inflammatory nature that would be created with an enzymatic fragmentation. 0,25% of

The products of the invention may contain a mixture of HA with different molecular weights, sonicated or unsonicated.

In the creams there is 0.25% of sonicated HMW, while in the serum there is 0.15% of High molecular weight (HMW) sonicated HA and 0.1% of LMW-HA.

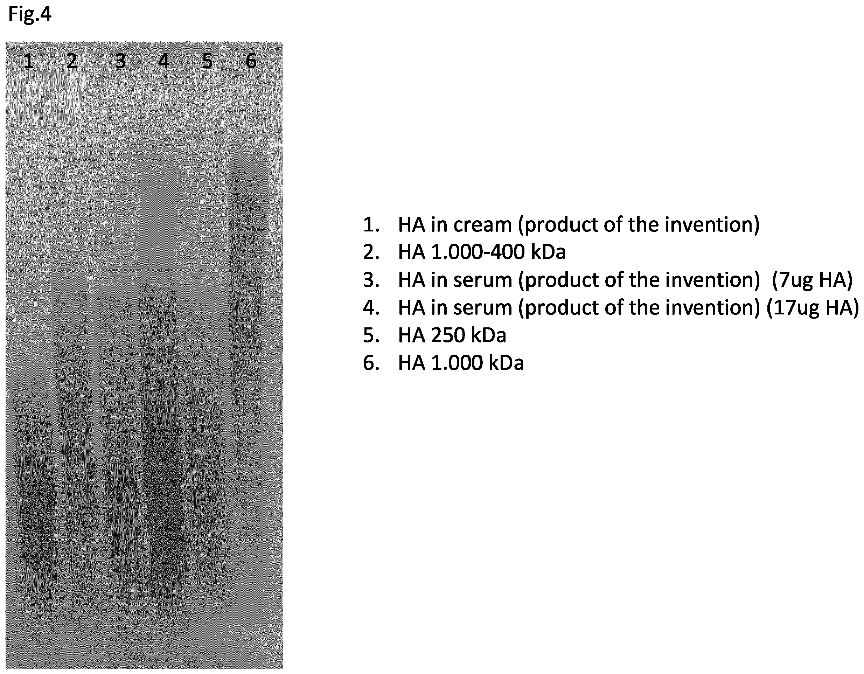

To verify the fragmentation of the HA, it was loaded onto an 1% agarose gel with reference samples and performed an electrophoretic run. (Figure 4).

Each sample distributes to form a smear. The smear is composed of HA fragments with different lengths: the upper part coincides with larger fragments which, being more voluminous, flow less inside the gel, while the lower part of the smear comprises smaller fragments. For example, the smear of 1000 kDa HA (sample 6) remain on the top of the gel.

The HA in Blast creams (sample 1) has a similar smear of the HA 250 kDa standard marker (sample 5).

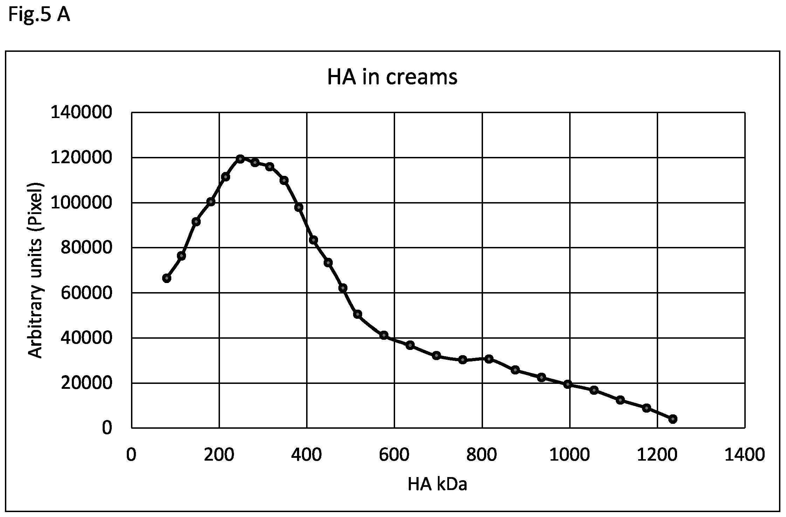

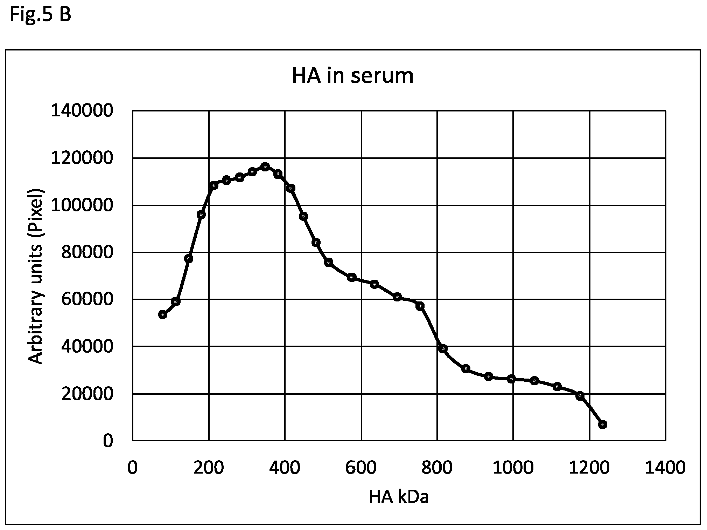

To obtain a more precise information on the distribution status of the HA fragments, the smear of the HA in Blast creams (sample 1) and HA in Blast serum (sample4) were divided into squares of the same size and the colour intensity of each interval was quantified (in pixel with imaged program) in relation with the standards. The more intense the colour, the greater the quantity of the fragment. The values were entered into a graph and percentages have been obtained. (Figure 5).

The molecular weight of HA in creams and serum is distributed according to a gaussian curve characterized by both medium-high and medium-low molecular weight HA.

The composition of HA in creams and HA in serum is described in the Table 4.

**Table 4:**

| **HA dimensions** | **HA in creams (a product of the invention)** | **HA in serum (a product of the invention)** |
|---|---|---|
| **1200 -800 kDa** | 9% | 11 % |
| **800-400 kDa** | 32,5 % | 41 % |
| **400-200 kDa** | 34,5 % | 37 % |
| **200-80 kDa** | 15% | 11 % |

The distribution guarantees the advantage of both high-medium and low-medium molecular weight. The particular size of the polysaccharide allows it to perform its action both at the level of the epidermis (fragments larger than 500 kDa), giving the skin the characteristics of elasticity, hydration and softness, and at the level of the dermis (fragments smaller than 500 kDa), helping the physiological maintenance of the extracellular matrix and its anti-inflammatory action.

### Example 8.: HA + CONDITIONED MEDIUM: STABILITY AND PROTECTION

The cosmetic product of the present invention comes from the combination of hyaluronic acid (HA) and Concentrated Conditioned Medium (CCM).

To provide evidence on how the quality and stability of an ingredient varies in time under the influence of environmental factors (temperature, humidity, light) and other components/ingredients, a stability test was performed.

In this experiment, stability of HA was evaluated in the presence of the CCM, the concentrate conditioned medium obtained from human fibroblasts cultured in vitro. The state of integrity of HA was evaluated with gel electrophoresis after 14 days.

The results clearly state that there are no differences in terms of magnitude between HA alone and HA mixed with CCM: the polysaccharide is the same size, no small fragments are formed and there are no visible aggregates. It is concluded that the CCM does not affect the integrity of the HA.

The stability of sonicated HA fragments is important for them to carry out their action both at the level of the epidermis and at the level of the dermis. In order to move into the epithelial layer, HA is subject to a series of interactions with the surrounding environment. Hyaluronidase, a powerful inhibitor of HA action which catalyzes the degradation of the polypeptide, resides in the skin. If on the one side its action can be advantageous for a high molecular weight HA (more than 1000 kDa) because it breaks it into smaller pieces that are more absorbable by the skin, on the other hand random degradation leads to the formation of small fragments of HA which may result pro-inflammatory.

To understand how CCM can help in the presence of this enzyme which is physiologically present in the skin, an enzymatic assay has been prepared. This test aims to mimic the physiological situation of the epidermis.

The purpose of this study was to obtain quantitative and not only qualitative data, in order to demonstrate that the presence of CCM has the ability to stabilize HA from the attack of hyaluronidases.

This test exploits the ability of long-chain hyaluronic acid to create turbidity in a solution containing albumin. Turbidity is a function of the HA concentration. The greater the turbidity, the greater the presence of HA. If the HA is cut by hyaluronidase, it is no longer able to form a network with albumin and the solution appears transparent. The decrease in turbidity is linked to the fragmentation of HA by the enzyme.

**Table 5: Enzyme competition assay with Hyaluronidase**

| Test with 5µg | OD (1) | OD (2) | OD (3) | OD average | OD average - blank | HA not digested | µg HA - µg HA in CCM | % of CCM protection of HA | % of enzyme degradation of HA |
|---|---|---|---|---|---|---|---|---|---|
| HA | 0.0652 | 0.0653 | 0.0634 | 0.0646 | 0.0203 | 4.348 | | | |
| HA + Hyase | 0.0453 | 0.0456 | 0.0445 | 0.0451 | 0.0008 | 0.109 | | 2.5 | 97.5 |
| HA + CCM Lot 1 + Hyase | 0.0732 | 0.0743 | 0.0728 | 0.0734 | 0.0291 | 6.261 | 4.21 | 96.83 | 3.17 |
| HA + CCM Lot 1 | 0.0753 | 0.0752 | 0.0746 | 0.075 | 0.0307 | 6.609 | 4.558 | | |
| CCM Lot 1 + Hyase | 0.0517 | 0.0524 | 0.0581 | 0.0541 | 0.0097 | 2.051 | 0 | | |
| Blank (only buffer enzyme) | 0.0447 | 0.0451 | 0.0432 | 0.0443 | 0 | | | | |

The data obtained and shown in Table 5 indicates that HA alone in the presence of the enzyme was almost completely degraded (97,5%), while HA in combination with CCM was minimally degraded (3,17%).

The competitive assay demonstrated the protective capacity of CCM towards HA when attacked by the enzyme.

The protective action of the CCC in favour of the stability of HA may be due to the sum of several factors. In fact, it appears that there are hyaluronidase inhibitors and/or antioxidants in the CCM which lead to less fragmentation of the HA. Furthermore, it is plausible that in CCM there are small enzymatic proteins capable of chemically modifying the structure of the polysaccharide making it more stable.

From the two tests performed, it is concluded that the sonicated HA has a size suitable for both epidermal and dermal action, and that it is able to interact with the proteins of the CCM (through hydrophobic interactions (charge-charge) and/or hydrogen bonds) forming a new compound with new characteristics, that is, more stable and less sensitive to the action of hyaluronidase. The interaction between HA and proteins was also exploited for transporting proteins at the sub-epithelial level. The result is an intradermal action of the mix.

Besides these data highlights the robustness and effectiveness of cosmetic product of the invention.

### Example 9.: HA + CONDITIONED MEDIUM: STABILITY AND PROTECTION: In vitro EFFICACY

A wound healing assay is an *in vitro* technique for probing collective cell migration. It is also called a scratch assay because it is done by making a scratch on a cell monolayer and capturing images at the beginning and at regular intervals during cell migration to close the wound and comparing the images to quantify the migration rate of the cells. The scratch assay is a great tool to study cell migration since this mechanism is involved in many different physiological aspects. Cell migration plays a huge role in re-epithelialization of the skin and so the study of cell migration can provide advancements in understanding non-healing wounds.

This assay was used to verify the ability of fragmented HA with an additional component CCM to promote the natural cellular migration of fibroblasts or keratinocytes miming the healing of a wound.

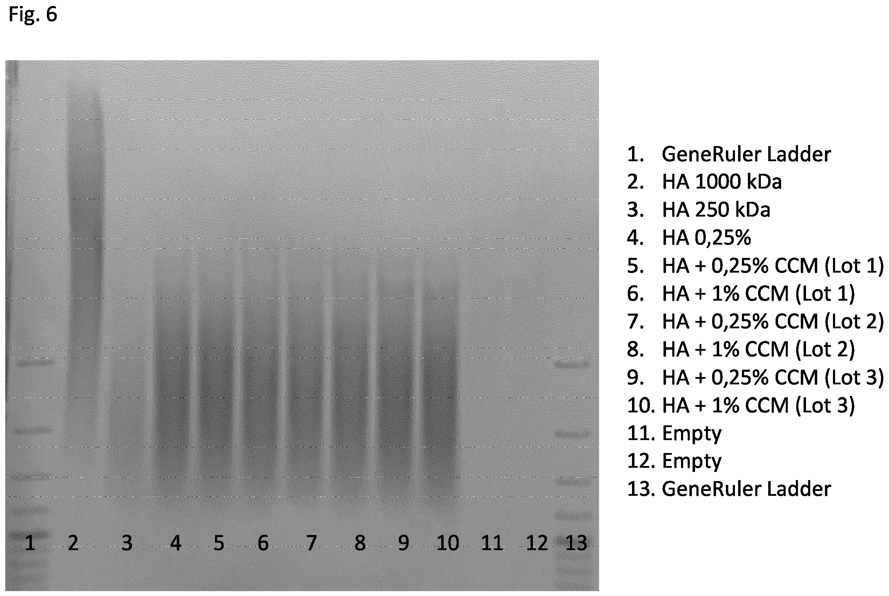

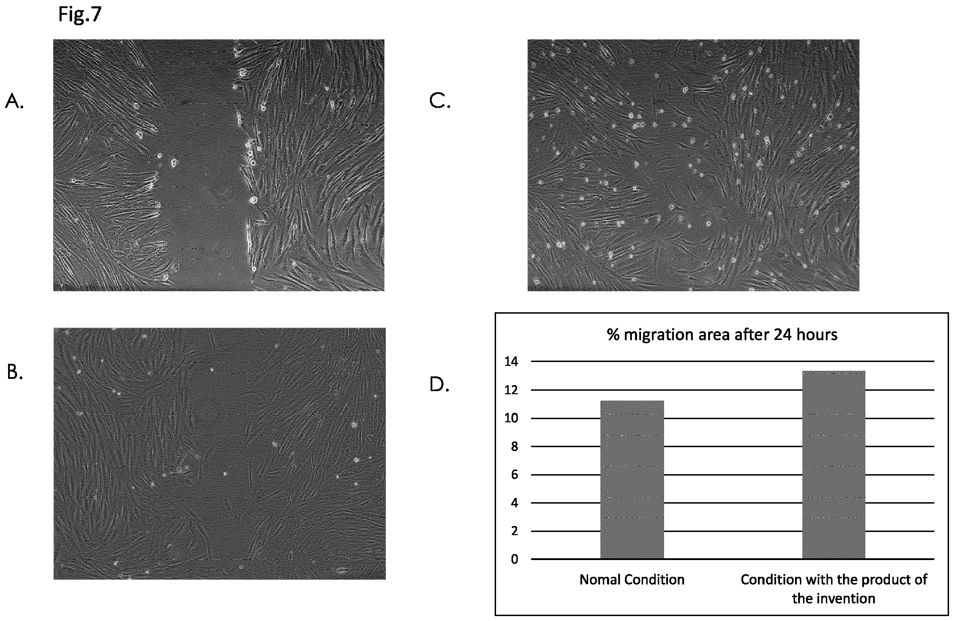

On the first day, a scratch is made on the cell monolayer. The cells are incubated with essential medium to mimic a physiologic condition or with a combination of HA and CCM. The scratch appears as a cell free space and is photographed in the same position at time 0 and after 24 hours. During this time the cells migrate into empty space trying to close the gap. Image analysis is performed using the TScratch program and the % of cell migration area is calculated (Figures 6 and 7).

Fibroblasts (Figures 6A-D) and keratinocytes (Figures 7A-D) incubated with the cosetic product of the invention were faster to close the space of injury, when compared with cells treated with essential medium. The cosmetic product facilitated the natural cell migration after an injury.

From the scratch test it was deduced that the two components together have a strengthening and synergic effect with respect to their single action: the HA mechanical visco-elastic properties support the physiological derma regeneration and the combination with CCM shows a stabilized action on HA.

### Example 10.: PRODUCTS INCI

The following tables show the components of some of the cosmetic products of the invention.

Cream Rich (Table 6), Cream Light (Table 7), Serum (face and/or body) (Table 8) and Hair Serum (Table 9).

**Table 6:**

| **Product** | **Ingredients** | **Function** | **%** |
|---|---|---|---|
| **Cream Rich** | SODIUM HYALURONATE | Functional ingredient | 0,250 |
| | Concentrated Conditioned Medium | Functional ingredient/Stabilizer of HA | 0,250 |
| | 3-O-ETHYL ASCORBIC ACID | Antioxidant | 0,170 |
| | AQUA | Solvent | 73,230 |
| | CAPRYLIC/CAPRIC TRIGLYCERIDE | Skin conditioning/Perfuming | 6,000 |
| | CETEARYL ETHYLHEXANOATE | Skin conditioning/Emollient | 4,000 |
| | DIISOSTEAROYL POLYGLYCERYL-3 DIMER DILINOLEATE | Skin conditioning/Emollient | 3,000 |
| | ISONONYL ISONONANOATE | Antistatic/Skin conditioning/Emollient | 2,500 |
| | CYCLOPENTASILOXANE | Skin conditioning/Emollient | 2,375 |
| | OCTYLDODECANOL | Skin conditioning/Emollient/Perfuming | 2,000 |
| | GLYCERIN (heptahydrate) | Humectant/Viscosity controlling | 2,000 |
| | MAGNESIUM SULFATE | Bulking/Viscosity controlling | 1,000 |
| | PHENOXYETHANOL | Preservative | 0,800 |
| | CERA ALBA | Emulsyfing/Emollient | 0,500 |
| | BUTYROSPERMUM PARKII BUTTER | Skin conditioning/ Viscosity controlling | 0,500 |
| | C12-15 ALKYL ETHYLHEXANOATE | Skin conditioning/Emollient | 0,500 |
| | HYDROGENATED CASTOR OIL | Skin conditioning/ Viscosity controlling/emollient | 0,500 |
| | IMIDAZOLIDINYL UREA | Preservative | 0,300 |
| | CYCLOHEXASILOXANE1 | Skin conditioning | 0,125 |

**Table 7:**

| **Product** | **Ingredients** | **Function** | **%** |
|---|---|---|---|
| **Cream Light** | AQUA | Solvent | 86,180 |
| | HEPTYL UNDECYLENATE | Skin conditioning/Emollient | 5,000 |
| | METHYLGLUCOSE SESQUISTEARATE | Surfactant, Emulsifying | 2,000 |
| | POLYGLYCERYL-3 METHYLGLUCOSE DISTEARATE | Surfactant, Emulsifying | 1,500 |
| | SIMMONDSIA CHINENSIS SEED OIL | Skin conditioning/Emollient | 1,000 |
| | BUTYROSPERMUM PARKII BUTTER | Skin conditioning/ Viscosity controlling | 1,000 |
| | STEARYL/OCTADODECYL CITRATE CROSSPOLYMER | Humectant | 0,800 |
| | CAPRYLYL GLYCOL | Skin conditioning/Emollient | 0,700 |
| | ACRYLATES/C10-30 ALKYL ACRYLATE CROSSPOLYMER | Emulsion Stabilizing, Film Forming, Viscosity controlling | 0,300 |
| | Concentrated conditioned medium | Functional ingredient/Stabilizer of HA | 0,250 |
| | SODIUM HYALURONATE | Functional ingredient | 0,250 |
| | ARGININE | Buffering, Skin conditioning | 0,200 |
| | IMIDAZOLIDINYL UREA | Preservative | 0,200 |
| | GLYCERYL CAPRYLATE | Skin conditioning/Emollient | 0,200 |
| | 3-O-ETHYL ASCORBIC ACID | Antioxidant | 0,170 |
| | XANTHAN GUM | Gel Forming, Emulsion Stabilizing, , Viscosity controlling | 0,150 |
| | ETHYLHEXYLGLYCERIN | Skin conditioning | 0,100 |

**Table 8:**

| **Product** | **Ingredients** | **Function** | **%** |
|---|---|---|---|
| **Serum** | SODIUM HYALURONATE | Functional ingredient | 0,250 |
| | Concentrated Conditioned Medium | Functional ingredient/Stabilizer of HA | 0,250 |
| | 3-O-ETHYL ASCORBIC ACID | Antioxidant | 0,170 |
| | AQUA | Solvent | 96,140 |
| | PHENOXYETHANOL | Preservative | 0,830 |
| | LACTIC ACID | Buffering/Humectant | 0,600 |
| | SODIUM POLYACRYLOYLDIMETHYL TAURATE | Emulsion stabilizing/viscosity controlling | 0,600 |
| | SODIUM LACTATE | Buffering/Humectant/Keratolytic | 0,400 |
| | IMIDAZOLIDINYL UREA | Preservative | 0,160 |
| | SODIUM HYDROXIDE | Buffering/denaturant | 0,400 |
| | BUTYLENE GLYCOL | Humectant/skin conditioning/viscosity controlling | 0,0005800 |
| | HYALURONIC ACID | Functional ingredient | 0,0000720 |
| | SILANETRIOL | Conditioning | 0,0000480 |
| | SORBIC ACID | Preservative | 0,0000480 |
| | CITRIC ACID | Buffering/chelating | 0,0000480 |

**Table 9:**

| **Product** | **Ingredients** | **Function** | **%** |
|---|---|---|---|
| **Hair Serum** | SODIUM HYALURONATE | Functional ingredient | 0,250 |
| | Concentrated Conditioned Medium | Functional ingredient/Stabilizer of HA | 0,250 - 0,375 - 0,5 - 1 |
| | AQUA | Solvent | 98,399 |
| | PHENOXYETHANOL | Preservative | 0,800 |
| | SODIUM POLY ACRYLOYLDI METHYL TAURATE | Emulsion stabilising/viscosity controlling | 0,100 |
| | IMIDAZOLIDINYL UREA | Preservative | 0,200 |
| | BUTYLENE GLYCOL | Humectant/skin cond itioning/viscosity controlling | 0,0003500 |
| | HYALURONIC ACID | Functional ingredient | 0,0000450 |
| | SILANETRIOL | Conditioning | 0,0000300 |
| | SORBIC ACID | Preservative | 0,0000300 |
| | CITRIC ACID | Buffering/chelating | 0,0000300 |

From the above description and the above-noted examples, the advantage attained by the cosmetic product described and obtained according to the present invention are apparent.

## Claims

1. A cosmetic product comprising:
- a concentrate comprising at least one growth factor and at least one cytokine, secreted from a fibroblast cell culture, and
- sonicated hyaluronic acid, having a mean molecular weight in the range from 80kDa to 1200kDa;
wherein said concentrate is obtainable by a process comprising the steps of:
a. Providing a fibroblast cell culture in a cell culture medium;
b. Stimulating the release of at least one growth factor and at least one cytokine into the cell culture medium of step a. to obtain a conditioned cell culture medium;
c. Isolating the conditioned cell culture medium of step b. from the cell culture, to obtain an isolated conditioned cell culture medium;
d. Concentrating the isolated conditioned cell culture medium of step c.

2. The cosmetic product according to claim 1, wherein said step b. of stimulating the release of at least one growth factor and at least one cytokine into the cell culture medium is carried out by subjecting said fibroblast cell culture to a step of nutrient deprivation.

3. The cosmetic product according to any one of claims 1 or 2, wherein said fibroblasts are isolated from a tissue sample chosen from the group consisting of skin and umbilical cord.

4. The cosmetic product according to claim 3, wherein said fibroblasts are isolated from said tissue sample by digestion with a protease, and subsequently with a collagenase.

5. The cosmetic product according to any one of claims 1 to 4, wherein said concentrate comprises a mixture of growth factors, cytokines and matrix proteins.

6. The cosmetic product according to any one of claims 1 to 5, wherein said concentrate comprises a growth factor chosen from the group comprising: Vascular Endothelial Growth Factor (VEGF), Transforming Growth Factor β (TGF-β) and Fibroblast Growth Factors (FGFs), Platelet-Derived Growth Factor (PDGF), Granulocyte-Colony Stimulating Factor (GCSF), Keratinocyte Growth Factor (KGF), Transforming Growth Factor Beta-3 (TGF beta 3), Nerve Growth Factor Beta (NGF beta), Sirtuin 1 (SIRT 1), Sirtuin 2 (SIRT2), Tissue Inhibitor for Metalloproteinase 1 (TIMP 1), Macrophage Colony Stimulating Factor 1 (M-CSF or CSF 1), Stem Cell Factor (SCF), Insulin-Like Growth Factor (IGF-1), Insulin-Like Growth Factor 2 (IGF 2), Interleukin 10 (IL-10) and Hepatocyte Growth Factor (HGF).

7. The cosmetic product according to any one of claims 1 to 6, wherein said sonicated hyaluronic acid comprises sonicated hyaluronic acid fragments have a size from 1200 to 800 kDa (5-20%), from 800 to 400 kDa (30-40%), from 400 to 200 kDa (30-40%) and from 200 to 80 kDa (5-20%).

8. The cosmetic product according to any one of claims 1 to 7, wherein said cosmetic product is in the form of a cream or a serum and further comprises cosmetically acceptable excipients.

9. Use of the cosmetic product according to any one of claims 1 to 8, for promoting tissue regeneration.

10. Use of the cosmetic product according to any one of claims 1 to 8, for improving skin texture.

11. Use of the cosmetic product according to any one of claims 1 to 8, for reducing skin wrinkles.

12. Use of the cosmetic product according to any one of claims 1 to 8, for reducing scars.

13. Use of the cosmetic product according to any one of claims 1 to 8, for reducing skin irritation and blemishing.
